# EUROPÄISCHE PATENTSCHRIFT

(11) **EP 2 753 254 B1**
(45) Veröffentlichungstag und Bekanntmachung des Hinweises auf die Patenterteilung: **06.09.2017**
(21) Anmeldenummer: 12737556.6
(22) Anmeldetag: 19.07.2012
(51) Int. Cl.: A61B 17/70, A61B 17/00

(54) **POLYAXIALE PEDIKELSCHRAUBE MIT PROVISORISCHER FIXIERUNG**
POLYAXIAL PEDICLE SCREW HAVING PROVISIONAL FASTENING
VIS PÉDICULAIRE POLYAXIALE À FIXATION TEMPORAIRE

(30) Priorität: 06.09.2011 DE 102011053295
(43) Veröffentlichungstag der Anmeldung: 16.07.2014
(73) Patentinhaber: Aesculap AG, 78532 Tuttlingen (DE)
(72) Erfinder: KRÜGER, Sven, 78647 Trossingen (DE)
(74) Vertreter: Winter, Brandl, Fürniss, Hübner, Röss, Kaiser, Polte - Partnerschaft mbB
(86) Internationale Anmeldenummer: PCT/EP2012/064207
(87) Internationale Veröffentlichungsnummer: WO 2013/034351

(56) Entgegenhaltungen:
- EP-A1- 2 502 594
- WO-A2-2005/058141
- US-A1- 2005 131 408
- US-A1- 2009 228 053

## Beschreibung

Die vorliegende Erfindung betrifft allgemein eine polyaxiale Pedikelschraube mit provisorischer/vorläufiger Fixierung gemäß dem Oberbegriff des Patentanspruchs 1, ein Hilfsmittel/Werkzeug zur provisorischen Fixierung der polyaxialen Pedikelschraube sowie ein Stabilisierungssystem bestehend aus der erfindungsgemäßen Pedikelschraube, dem erfindungsgemäßen Hilfsmittel sowie vorzugsweise einem Längsträger.

Pedikelschrauben dienen prinzipiell zur dorsalen Stabilisierung der Wirbelsäule bei Frakturen, Tumoren, Entzündungen, Deformitäten und degenerativ bedingten Instabilitäten mittels transpedikulärer Verschraubung. Dabei werden Pedikelschrauben in den Pedikeln jeweils benachbarter Wirbel platziert, worauf eine winkelstabile Verbindung zwischen den jeweils axial übereinander sich anordnenden Pedikelschrauben und einem axial sich erstreckenden Längsträger oder Steg geschaffen wird. Die Pedikelschrauben und Längsträger bilden dabei ein Wirbel-Stabilisierungssystem.

Hierfür hat eine Pedikelschraube in der Regel einen axialen, schaftartigen Außengewindeabschnitt, an den sich schraubenkopfseitig eine sogenannte Tulpe anschließt. Diese bildet konstruktiv eine U-förmig längs geschlitzte/getunnelte Aufnahmehülse mit Innengewinde, wobei die beiden sich radial gegenüberliegenden Längsschlitze jeweils einen Schlitzspalt vorbestimmter Spaltbreite definieren. In die parallel zueinander verlaufenden Längsschlitze ist der Längsträger quer eingelegt, der mittels eines Verriegelungselements beispielsweise einer Madenschraube oder Gewindemutter, die in das Innengewinde eingedreht ist, fixiert wird.

Grundsätzlich werden zwei Grundarten von Pedikelschrauben unterschieden, nämlich monoaxiale sowie polyaxiale Pedikelschrauben. Im Fall einer monoaxialen Pedikelschraube sind der Außengewindeabschnitt bzw. Schaft und die Tulpe einstückig miteinander ausgebildet, derart, dass sie grundsätzlich fest miteinander verbunden, beispielsweise verschweißt oder verlötet sind. Eine polyaxiale Pedikelschraube hat hingegen einen als separates Schaftbauteil gefertigten Außengewindeabschnitt mit einem zumeist kugelförmigen oder (semi-) sphärischen Schraubenkopf, der von der hülsenförmigen Tulpe relativ verschwenkbar umgriffen und gleichzeitig im Übergangsbereich zwischen Kopf und Schaft hintergriffen wird. Auf diese Weise kann die Tulpe nach Versenken des Außengewindeabschnitts im Pedikelkanal eines Wirbels relativ zum Schaft verschwenkt und/oder verdreht werden, um eine gewünschte Lage und Ausrichtung im Wesentlichen unabhängig zur Ausrichtung des Schafts zu erhalten. Die Hinterschneidung verhindert dabei, dass die Tulpe vom Schaftkopf abgezogen werden kann. Anschließend wird die Tulpe mittels der Madenschraube bei zwischengelagertem Steg (Ein-Schrauben-Prinzip) oder durch eine zusätzliche Schraube/Schraubenmutter (Mehr-Schrauben-Prinzip) am Schraubenkopf lagefixiert.

Pedikelschrauben werden von einem Operateur in den Pedikelkanal eines Wirbels eingesetzt bzw. durch Verschrauben verankert. Dabei richtet der Operateur die Schrauben aufgrund der Orientierung des Pedikelkanals aus. Wenn die Schrauben gesetzt sind, wird der vorstehend genannte Längsträger oder Steg der richtigen Länge ausgewählt und ggf. in seiner Längskrümmung an die Pedikelschrauben sowie deren jeweilige Position angepasst. Der Längsträger soll dabei in den Tulpen der Pedikelschrauben platziert werden.

Die polyaxialen Pedikelschrauben zeichnen sich nunmehr dadurch aus, dass die Tulpe/Aufnahmehülse zunächst beweglich zum Schraubenschaft gelagert ist, sodass die Aufnahmehülse eine andere Ausrichtung annehmen kann, als der Schaft. Dies erleichtert es dem Operateur, den Längsträger in die Aufnahmehülse lateral einzusetzen/einzuführen. Ist der Operateur mit dem Sitz des Längsträgers und der Aufnahmehülse zufrieden, verriegelt er die Pedikelschraube mittels des Verriegelungselements vorzugsweise der Madenschraube (prinzipiell auch Setzschraube beliebiger Ausgestaltung genannt).

Die Polyaxialverriegelung (Lagefixation der Aufnahmehülse bezüglich des Schafts) sowie die Längsträgerverklemmung werden im Fall des vorstehend genannten Ein-Schrauben-Prinzips beim Anziehen der einen Setzschraube in einem einzigen Montageschritt verriegelt.

Bei Polyaxialschrauben ist es aufgrund der Beweglichkeit (Gelenk) zwischen dem Schraubenschaft und der Aufnahmehülse während des Implantierungsvorgangs in der Regel nicht möglich, Kräfte von der Aufnahmehülse aus in den Wirbel einzuleiten, um diesen beispielsweise manipulieren zu können. Dies ist insbesondere bei Repositionsmanövern im Fall von Frakturen oder Spondylolisthesen und zum Teil bei Kompressionen oder Distraktionen jedoch erforderlich. Hierfür werden daher die monoaxialen Pedikelschrauben verwendet, bei welchen die Aufnahmehülse zum Schraubenschaft starr ist. Diese monoaxialen Pedikelschrauben haben jedoch wiederum den Nachteil, dass der Längsträger nur schwer in die Tulbe/Aufnahmehülse mehrerer monoaxialen Pedikelschrauben lateral eingeführt werden kann.

Aus dem Stand der Technik, beispielsweise gemäß der EP 2 301 458 A1 ist eine polyaxiale Pedikelschraube gemäß dem vorstehend erwähnten Ein-Schrauben-Prinzip bekannt bestehend aus einem Schaftbauteil mit Außengewinde und sphärischem Kopf sowie einer U-förmig längs geschlitzten Aufnahmehülse (Tulpe) für einen Längsträger/Steg. Die Aufnahmehülse hat im Axialbereich hin zur Öffnung der Längsschlitze ein Innengewinde, in das eine Madenschraube eindrehbar ist und im Axialbereich hin zum jeweiligen Schlitzgrund einen radial nach innen gerichteten Umfangsvorsprung oder Absatz. Ferner ist in die Aufnahmehülse eine Art Kolben oder Stempel mittels eines Sprengrings eingesetzt und dadurch gegen ein Herausfallen gesichert.

Zur Montage der aus der EP 2 301 458 A1 bekannten polyaxialen Pedikelschraube wird zunächst die Aufnahmehülse vom distalen (dem Schaftkopf gegenüberliegenden) Ende des Schaftbauteils her über dieses gestreift, solange, bis der radiale Innenabsatz der Aufnahmehülse gegen den Schaftkopf (an dessen Unterseite) anschlägt. Anschließend wird der Stempel in die Aufnahmehülse eingedrückt (d.h. auf der Oberseite des Schaftkopfs), sodass der umfangsseitig zwischen Aufnahmehülse und Stempel sich anordnende Sprengring in entsprechende Umfangsnuten am Stempel und an der Aufnahmehülse einschnappt und die beiden Bauteile axial aneinander hält. Der Schaftkopf ordnet sich somit zwischen dem Absatz der Aufnahmehülse und dem Stempel (d.h. unterhalb des Stempels) an.

Sobald die Pedikelschraube in einen Wirbel eingedreht und fest darin verankert ist, wird ein Längsträger in den U-förmigen (Doppel-) Schlitz der Aufnahmehülse (oberhalb des Stempels) eingeführt, wobei sich die Aufnahmehülse relativ zum verankerten Schaftbauteil drehen und verschwenken kann. Auf diese Weise ist es einem Operateur möglich, die Aufnahmehülse entsprechend der Ausrichtung des Längsträgers anzupassen. Sobald die geeignete Relativlage der Aufnahmehülse eingestellt ist, wird die Madenschraube in die Aufnahmehülse soweit einschraubt, bis diese den Längsträger gegen den Stempel anlegt und diesen weiter in Axialrichtung der Aufnahmehülse gegen den Schaftkopf drückt. Auf diese Weise kann durch Festziehen der einzigen Madenschraube (Verriegelungselement) das gesamte Pedikelschrauben-Längsträger-System (Wirbel-Stabilisierungssystem) in der eingestellten Positionierung fixiert/verriegelt werden.

Die US 2008/02155100 A1 offenbart beispielsweise eine polyaxiale Pedikelschraube gemäß dem vorstehend genannten Mehr-Schrauben-Prinzip. Auch diese Pedikelschraube hat einen schaftartigen Außengewindeabschnitt mit einem integralen Schaftkopf an einem proximalen Ende des Schafts. Der Schaftkopf ist von einer Aufnahmehülse frei dreh- und schwenkbar umgeben, in der ebenfalls ein Innengewinde ausgebildet ist und die zwei U-förmige, sich radial gegenüberliegende Längsschlitze für einen Längsträger aufweist.

In die Aufnahmehülse ist ein Kolben/Stempel axial verschieblich eingesetzt, der ebenfalls einen U-förmigen Längsschlitz in etwa mit den Schlitzbreiten-Abmessungen der Längsschlitze in der Aufnahmehülse aufweist. Die hierbei entstehenden Flanken am U-förmigen Längsschlitz des Stempels sind in Axialrichtung so verlängert, dass sie über einen darin quer eingelegten Steg/Längsträger ragen. D.h. die Länge der Flanken des Stempels ist größer als der Durchmesser des quer eingelegten Stegs/Längsträgers.

Um die aus der US 2008/02155100 A1 bekannte Pedikelschraube zu montieren, wird in bekannter Weise die Aufnahmehülse/Tulpe über den Schaft gestreift, bis diese axial am Schaftkopf (unterseitig) schwenk- und drehbar anliegt. Hierauf wird der Stempel (oberhalb des Schaftkopfs) in die Aufnahmehülse eingesetzt und dessen U-förmiger Schlitz entsprechend den U-förmigen Schlitzen in der Aufnahmehülse ausgerichtet. Nachdem ein Längsträger/Steg in den U-förmigen Schlitz des Stempels (und hierbei zwangsläufig auch in die Schlitze der Aufnahmehülse) eingeführt ist, wird eine erste Schraube (Schraubenhülse), vorzugsweise eine Madenschraube, die zuvor in das Innengewinde der Aufnahmehülse eingedreht wurde, weiter festgezogen, um den Stempel unmittelbar gegen den Schaftkopf zu pressen und somit die Aufnahmehülse relativ zum Schaft lagezufixieren. Der Längsträger bleibt von der ersten Schraube jedoch zunächst unbeeinflusst, d.h. nicht fixiert. In dieser Montagesituation ist es möglich, eine Justierkraft über die Aufnahmehülse auf den Wirbel aufzubringen.

Die erste Schraube ist ebenfalls hülsenförmig und hat ein Innengewinde, in das eine zweite Schraube (Madenschraube) eingedreht ist. Mittels dieser zweiten Schraube wird nunmehr der Längsträger im Stempel fixiert, in dem die zweite Schraube relativ zur ersten Schraube gedreht wird und unmittelbar gegen den Steg drückt, um diesen im Stempel einzuklemmen. Damit ist die Polyaxialität der Pedikelschraube verriegelt und dann auch der Längsträger fixiert.

Weitere gattungsgemäße polyaxiale Pedikelschrauben sind beispielsweise auch aus US 2009/0228053 A1, US 2005/0131408 A1 oder WO 2005/058141 A2 bekannt.

EP 2 502 594 A1 offenbart eine polyaxiale Pedikelschraube sowie ein Fixationssystem mit einer entsprechenden polyaxialen Pedikelschraube. Die Pedikelschraube weist einen Aufnahmekopf mit einem transversalen U-förmigen Durchgang sowie einen (Schraub-)Schaft mit einem Schaftkopf auf. Ein Verriegelungseinsatz zur provisorischen Verriegelung kann über seitliche Öffnungen seitlich durch den Aufnahmekopf in diesen eingeführt werden. Über einen Zug-Druck-Mechanismus, wobei ein Druck auf den Verriegelungseinsatz und ein Zug auf den Aufnahmekopf aufgebracht wird, kann die Pedikelschraube mithilfe des zugehörigen Fixationssystems provisorisch fixiert/verriegelt werden. Die Flanken der Aufnahmehülse weisen axial verlaufende und verschnittene Nuten auf, über welche der Druckstößel eingeführt und Druck auf das Verriegelungselement aufgebracht werden kann.

Bei vielen Pedikelschrauben gemäß vorstehend beschriebenem Aufbau sind die Fixiermittel/Verriegelungselemente (Schrauben) im Wesentlichen selbsthemmend ausgeführt, um nach Implantierung kein ungewolltes Lösen der Längsträger von den Pedikelschrauben zu riskieren. Darüber hinaus sind die Fixationskräfte zwischen Pedikelschraube und Längsträger beträchtlich, da das gesamte System großen Belastungen standhalten muss, ohne dass sich die eingestellte Lagebeziehung zwischen Schaft, Hülse und Längsträger verändern darf. Daraus ergibt sich, dass die Klemmkräfte zwischen dem Schaftkopf und der Aufnahmehülse ebenfalls sehr hoch sind, sodass der dazwischen sich ausbildende Reibschluss häufig selbst dann nicht frei kommt, wenn das Fixiermittel gelöst ist. Diese Notwendigkeiten verursachen indessen auch Probleme während des Implantierungsvorgangs.
- Solange die Aufnahmehülse nicht fest (reibschlüssig) mit dem Schaftkopf verbunden ist, kann über diese keine Justierkraft auf den Wirbel übertragen werden. D.h., beispielsweise im Fall einer polyaxialen Pedikelschraube nach dem Ein-Schrauben-Prinzip müsste demnach zunächst ein Längsträger eingelegt und dann das Verriegelungselement, vorzugsweise die Madenschraube festgezogen werden, um schließlich eine Justierkraft auf den Wirbel aufbringen zu können. Dies wäre jedoch technisch nicht sinnvoll.
- Hat ein Operateur erst einmal das Verriegelungselement (beispielsweise die Madenschraube) mit Kraft festgezogen und ergibt sich ggf. nachträglich die Notwendigkeit einer Nachjustierung der Wirbelposition, ist die hierdurch festgelegte Lagebeziehung nicht, oder nur mit großem Aufwand wieder veränderbar. In anderen Worten ausgedrückt, müsste hierfür der Operateur die mit großer Kraft festgezogene(n) Madenschraube(n) entgegen deren Selbsthemmwirkung wieder lösen, ohne hierbei den im Wirbel bereits verankerten Außengewindeabschnitt mit zu lösen oder gar herauszubrechen. Selbst wenn die Madenschraube problemlos gelöst werden kann, hat sich zwischen dem Schaftkopf und der Aufnahmehülse ggf. ein selbsthemmender Reibschluss ausgebildet. Dieser könnte auch bei gelöster Madenschraube nur durch erheblichen Kraftaufwand (Schläge auf die Aufnahmehülse, etc.) freikommen. Des Weiteren wird durch das nachträgliche Lösen des Verriegelungselements dessen Selbsthemmwirkung ggf. beeinträchtigt, sodass die Funktionsfähigkeit der Pedikelschraube nicht mehr gewährleistet ist.

In sofern wäre es grundsätzlich vorteilhaft, wenn die Polyaxialität insbesondere im Fall des Ein-Schrauben-Prinzips quasi nur provisorisch fixierbar ist, um so die Polyaxialität und den Steg/Längsträger zumindest temporär unabhängig verriegeln zu können. In anderen Worten ausgedrückt wäre es sinnvoll, bei einer polyaxialen Pedikelschraube auch nach dem Ein-Schrauben-Prinzip mittels eines temporär einzusetzenden besonderen Hilfsmittels nur die Polyaxialität zu fixieren, ohne dass der Steg fixiert wird. Dies hätte den Vorteil, dass nunmehr auch die polyaxiale Pedikelschraube dafür geeignet wäre, vor Fixierung des Längsträgers entsprechend hohe Kräfte auf den Wirbel für dessen Manipulation während des Implantierungsvorgangs zu übertragen, ohne dass der innere Aufbau der Pedikelschraube wesentlich komplizierter wird.

An dieser Stelle sei darauf hingewiesen, dass unter dem Begriff "provisorisch" insbesondere mit Blick auf die nachfolgend beschriebene Erfindung nicht notwendiger Weise nur ein leichtes Anlegen/Verklemmen des Schaftkopfs gemeint sein soll, sondern insbesondere eine solche Verriegelungsmaßnahme zu verstehen ist, die temporär angewendet wird, sowie deren erzielbare Verriegelungseigenschaften wie die Einklemmkraft auf den Schaftkopf, etc. durchaus der dauerhaften Verriegelung entsprechen, dieser zumindest nahe kommen oder diese sogar übertreffen können. In anderen Worten ausgedrückt sollte die "provisorische" Verriegelung so dimensioniert sein, dass sie ein Verklemmen der Aufnahmehülse am Schaftkopf entsprechend dem dauerhaften Verriegelungselement bewirkt. In diesem Fall wäre zwar ggf. ein nachträgliches Lösen des Reibschlusses in Übereinstimmung mit dem Stand der Technik nur durch erhebliche Krafteinwirkung möglich, aber es bestünde wenigstens die Möglichkeit der Wirbelnachjustierung vor Fixierung des Längsträgers. Alternativ wäre es aber auch wünschenswert, die "provisorische" Verriegelung so zu dimensionieren, dass ausreichende Justierkräfte auf den Wirbel mittels der polyaxialen Pedikelschraube übertragbar sind, wobei der hierbei erzielte Reibschluss zwischen Schaftkopf und Aufnahmehülse wieder lösbar ist.

Indessen erweisen sich die polyaxialen Pedikelschrauben gemäß dem beschriebenen Stand der Technik als nachteilig. So kann insbesondere im Fall des Ein-Schrauben-Prinzips die Polyaxialität erst dann verriegelt werden, wenn der Längsträger in seiner Endposition sitzt (noch nicht fixiert). Ein Einsetzen des Längsträgers nach dem Verriegeln der Polyaxialität ist eigentlich nicht oder nur sehr schwer möglich und hätte einen deutlich komplizierteren Aufbau der Pedikelschraube zur Voraussetzung.

Angesichts der vorstehend geschilderten Problematik besteht eine Aufgabe der Erfindung darin, die Funktionalität einer polyaxialen Pedikelschraube zu erhöhen, insbesondere indem die Vorzüge einer monoaxialen Pedikelschraube mit denen einer polyaxialen Pedikelschraube kombiniert werden. Ein Ziel ist es, die Manipulierbarkeit eines Wirbels über die polyaxiale Pedikelschraube (gemäß beliebigem Ausführungsprinzip) zu ermöglichen, ohne dass die Einlegbarkeit des Längsträgers in die Tulpe über Gebühr erschwert wird. Des Weiteren sollte die polyaxiale Pedikelschraube keinen (wesentlich) komplizierteren Aufbau haben als der Stand der Technik.

Die genannte Aufgabe wird durch eine polyaxiale Pedikelschraube mit provisorischer/vorläufiger Fixierung (im Sinne vorstehender Definition) gemäß dem Patentanspruch 1, ein Hilfsmittel/Werkzeug zur provisorischen Fixierung (im Sinne vorstehender Definition) der polyaxialen Pedikelschraube sowie ein Stabilisierungssystem bestehend aus der erfindungsgemäßen Pedikelschraube und dem erfindungsgemäßen Hilfsmittel gelöst. Vorteilhafte Weiterbildungen der Erfindung sind im Wesentlichen Gegenstand der Unteransprüche.

Der Grundgedanke der Erfindung beruht im Wesentlichen darauf, die Fixation (das Verriegeln) der Polyaxialität provisorisch/temporär durch ein externes Hilfsmittel zu bewirken, das parallel zum eigentlichen (dauerhaften) Verriegelungselement der polyaxialen Pedikelschraube auf den Stempel und die Aufnahmehülse (vorzugsweise mit vergleichbarer Kraft) einwirkt, um diese gegeneinander vorzuspannen und dabei den zwischen Stempel und Aufnahmehülse sich anordnenden Schaftkopf (in Übereinstimmung mit dem eigentlichen Verriegelungselement) einzuklemmen. Der Stempel sowie die Aufnahmehülse sind demzufolge mit Eingriffselementen für das Hilfsmittel versehen/ausgerüstet, an denen das Hilfsmittel jeweils (kraftschlüssig) angreift, um eine entsprechende Vorspannkraft auf die betreffenden Elemente (Stempel und Aufnahmehülse) anzulegen. Das Eingriffselement am Stempel/Stempel-Eingriffselement ist dabei für das Anlegen einer Druckkraft und das Eingriffselement an der Aufnahmehülse/Aufriahmehülse-Eingriffselement für das Anlegen einer Zugkraft vorbereitet. Die Aufnahmehülse weist dabei einen beidseits offenen, insbesondere zylindrischen, Hülsenkörper auf, dessen Hülsenwand von einer proximalen Stirnseite her U-förmig aufgeschlitzt ist, wodurch sich zwei radial gegenüberliegende Flanken ergeben, wobei sich die Stempel-Eingriffselemente zwischen beiden Flanken befinden.

In anderen Worten ausgedrückt kann das (externe) Hilfsmittel erfindungsgemäß so gestaltet sein, dass es bei seiner wie vorstehend definierten temporären Anwendung das eigentliche Verriegelungselement mit Bezug auf die Fixierung der Polyaxialität zeitweise ersetzt oder simuliert und einen Reibschluss zwischen Schaftkopf und Aufnahmehülse erzeugt, der je nach gewünschter Einstellung gleich, größer oder geringfügig kleiner ist als der durch das dauerhafte Verriegelungselement erzielte Reibschluss. Das bedeutet also, dass der Begriff "temporär" nicht dahingehend verstanden werden soll, dass der mittels des Hilfsmittels erzielte Reibschluss zwingend wieder lösbar ist sondern nur, dass das Hilfsmittel zur provisorischen Erzeugung des (lösbaren oder nicht mehr lösbaren) Reibschlusses parallel zum dauerhaften Verriegelungselement vorübergehend eingesetzt wird.

Konstruktiv wird der vorstehende prinzipielle Grundgedanke im Wesentlichen dadurch umgesetzt, indem der Stempel derart ausgeformt ist, dass er zumindest abschnittsweise radial über das verwendete Verriegelungselement vorsteht bzw. freiliegt, wodurch sich an diesen radialen Abschnitten, die vom Verriegelungselement nicht abgedeckt/überlagert sind, Angriffspunkte für das Einleiten einer Druckkraft auf den Stempel parallel zum eigentlichen Verriegelungselement entstehen. Im Gegenzug ist die Aufnahmehülse mit Hinterschneidungen ausgebildet/versehen, die für die äußere Einleitung einer Zugkraft ausgelegt/geeignet sind.

Im Konkreteren hat die erfindungsgemäße polyaxiale Pedikelschraube einen geschraubten Schaftabschnitt zur Verankerung der Pedikelschraube in einem Wirbel, an dessen einem Axialende ein (sphärischer/semisphärischer) Schaftkopf ausgebildet ist. Der Schaftkopf ist mit einer Aufnahmehülse für einen Längsträger dreh- und/oder Schwenkbar gekoppelt, welche ein Fixiermittel zur wahlweisen Lagefixierung der Aufnahmehülse bezüglich des Schaftabschnitts aufweist. Das Fixiermittel hat einen in der Aufnahmehülse gelagerten sowie auf den Schaftkopf einwirkenden Stempel (Inlay) und ein auf den Stempel einwirkendes Verriegelungselement, wodurch der Stempel gegen den Schaftkopf vorspannbar ist und damit der Schaftkopf zwischen dem Stempel und der den Schaftkopf formschlüssig hintergreifenden Aufnahmehülse (reibschlüssig) einklemmt. Gemäß der Erfindung ist der Stempel mit vom Verriegelungselement nicht abgedeckten oder überlagerten Stempel-Eingriffselementen ausgebildet/versehen für das Einleiten einer (provisorischen/temporären) Druckkraft auf den Stempel parallel zum Verriegelungselement vorzugsweise unter Verwendung eines separaten, werkzeuggleichen Hilfsmittels, wobei die Aufnahmehülse einen beidseits offenen, insbesondere zylindrischen, Hülsenkörper aufweist, dessen Hülsenwand von einer proximalen Stirnseite her U-förmig aufgeschlitzt ist, wodurch sich zwei radial gegenüberliegende Flanken ergeben, wobei sich die Stempel-Eingriffselemente zwischen beiden Flanken befinden. Weiterhin sieht die Erfindung vor, dass der Stempel derart ausgeformt ist, dass er zumindest abschnittsweise radial über das Verriegelungselement vorsteht bzw. freiliegt, wodurch sich an diesen radialen Abschnitten, die vom Verriegelungselement nicht abgedeckt oder überlagert sind die Angriffspunkte (für das separate Hilfsmittel) entstehen.

Vorteilhaft kann es sein, wenn der Stempel zumindest an einem axialen (Stempel-) Abschnitt einen kreisabweichenden Grundriss, vorzugsweise einen ovalen Grundriss hat, wodurch jene Grundrissabschnitte mit großem Durchmesser radial über das Verriegelungselement vorragen und die Angriffspunkte enthalten. Alternativ hierzu ist es aber auch möglich, radial vorragende Laschen am Stempel auszuformen oder dem Stempel sogar ein Mehr-Eck-Profil zu geben, wodurch Grundrissbereiche entstehen, die radial über das Verriegelungselement, vorzugsweise ein Schraube/Madenschraube vorstehen. Auf diese Weise kann ein separates Hilfsmittel/Werkzeug zur provisorischen/temporären Fixierung zumindest der Polyaxialität am (noch nicht verriegelten) Verriegelungselement vorbeigeführt und mit dem Stempel in axialen Druckeingriff gebracht werden.

Vorzugsweise sind die Angriffspunkte durch axiale Zentrierlöcher markiert, die zur Aufnahme und Platzierung eines externen Hilfsmittels zur Druckbeaufschlagung des Stempels dienen. Somit kann das Hilfsmittel nicht vom Stempel abgleiten und die vorragenden Grundrissabschnitte des Stempels können vergleichsweise klein gestaltet sein.

Um eine ausreichend große provisorische/temporäre Fixationskraft auf den Stempel aufbringen zu können (vergleichbar zum eigentlichen Verriegelungselement) ist es vorteilhaft, wenn der Stempel und die Aufnahmehülse mittels des separaten Hilfsmittels gegeneinander vorspannbar sind. Zu diesem Zweck kann die Aufnahmehülse mit zumindest einem Aufnahmehülse-Eingriffselement ausgebildet oder versehen sein, über das eine äußere provisorische Zugkraft auf die Aufnahmehülse parallel zum Verriegelungselement aufbringbar ist. Vorzugsweise ist das Aufnahmehülse-Eingriffselement eine Hinterschneidung, die weiter vorzugsweise an der äußeren Mantelseite der Aufnahmehülse ausformt ist. Dies hat den Vorteil der radialen Verlagerung jenes Bauteils des Hilfsmittels, das eine Druckkraft auf den Stempel anlegen soll von jenem Bauteil, mit welchen eine (Gegen-) Zugkraft auf die Aufnahmehülse aufgebraucht wird. Damit können beide Bauteile des Hilfsmittels quasi ineinander gelegt werden, wodurch dieses sehr kompakt und auch stabil wird. Weiterhin betrifft die Erfindung auch ein Hilfsmittel zur provisorischen/temporären Fixierung der Polyaxialität einer Pedikelschraube, wie diese vorstehend beschrieben wurde. Das als ein separates Werkzeug vorgesehene Hilfsmittel hat ein erstes Bauteil vorzugsweise in Form eines Druckstößels für das drückende in Eingriff kommen mit den am Stempel vorgesehenen Angriffspunkten und ein zweites Bauteil vorzugsweise ein Zugbauteil, das relativ, vorzugsweise gegenläufig, zum ersten Bauteil bewegbar ist und ein Rast- oder Einhakelmittel hat, das mit der Aufnahmehülse, vorzugsweise an deren Eingriffselement in ziehenden Eingriff bringbar ist. Vorzugsweise ist das Zugbauteil eine den Druckstößel umgebende Hülse, an deren einem Axialende radial einwärts vorstehende Rastvorsprünge, Haken oder Leisten ausgeformt sind, um mit der Aufnahmehülse vorzugsweise an deren Eingriffselementen in lösbaren Rasteingriff zu kommen. Ferner ist die Hülse vorzugsweise auf einem den Rastvorsprüngen gegenüberliegenden Axialendabschnitt mit dem Druckstößel über eine Kupplung wirkverbunden. Diese Kupplung bewirkt, dass eine Druckkraft vom Drückstößel in eine Zugkraft von der umgebenden Hülse umgewandelt wird, wodurch sich ein provisorischer/temporärer geschlossener Kraftfluss zwischen Stempel, Schaftkopf, Aufnahmehülse und separates Hilfsmittel ergibt. Die Verankerung der Pedikelschraube im Wirbel bleibt hiervon unberührt. Die Erfindung betrifft auch ein Stabilisierungssystem das folgende Bestandteile hat:
- eine polyaxiale Pedikelschraube, wie diese vorstehend beschrieben wurde und
- ein manuell betätigbares Hilfsmittel, wie dieses vorstehend beschrieben wurde. Vorzugsweise hat das Stabilisierungssystem der Erfindung des Weiteren
- einen Längsträger, der in einem Querschlitz der Aufnahmehülse einlegbar ist, wobei zumindest das erste Bauteil und vorzugsweise auch das zweite Bauteil des Hilfsmittels so gestaltet ist/sind (vorzugsweise mit U-förmigen Längsschlitzen), dass es/sie den Längsträger umgreift (en), derart, dass der Längsträger auch dann noch eingesetzt werden kann, wenn das Hilfsmittel bereits angesetzt und aktiviert ist.

Die Erfindung wird nachstehend anhand eines bevorzugten Ausführungsbeispiels unter Bezugnahme auf die begleitenden Figuren näher erläutert.
Fig. 1 zeigt die Perspektivenansicht einer polyaxialen Pedikelschraube gemäß einem bevorzugten Ausführungsbeispiel der Erfindung,
Fig. 2 zeigt die Pedikelschraube von Fig. 1 in Draufsicht,
Fig. 3 zeigt die Pedikelschraube von Fig. 1 teilweise aufgebrochen,
Fig. 4 zeigt die Perspektivenansicht eines Stabilisierungssystems gemäß einem bevorzugten Ausführungsbeispiel der Erfindung bestehend aus der Pedikelschraube gemäß der Fig. 1 bis 3 und einem werkzeugartigen externen Hilfsmittel,
Fig. 5 zeigt eine weitere Perspektivenansicht des erfindungsgemäßen Stabilisierungssystems mit Hilfsmittel in Funktionsposition,
Fig. 6 zeigt eine Perspektivenansicht des erfindungsgemäßen Stabilisierungssystems mit Hilfsmittel in Funktionsposition und eingelegtem Längsträger,
Fig. 7 zeigt eine Perspektivenansicht des erfindungsgemäßen Stabilisierungssystems gemäß Fig. 6 zusätzlich mit eingesetztem Verriegelungselement,
Fig. 8 zeigt das Stabilisierungssystem in fertig montiertem Zustand sowie abgenommenem Hilfsmittel,
Fig. 9a-9b zeigt eine Perspektivenansicht des Hilfsmittels gemäß der Erfindung ohne Kupplung und
Fig. 10 zeigt eine Seitenansicht des Hilfsmittels gemäß der Fig. 9 mit Kupplung in Form einer Wellenmutter.

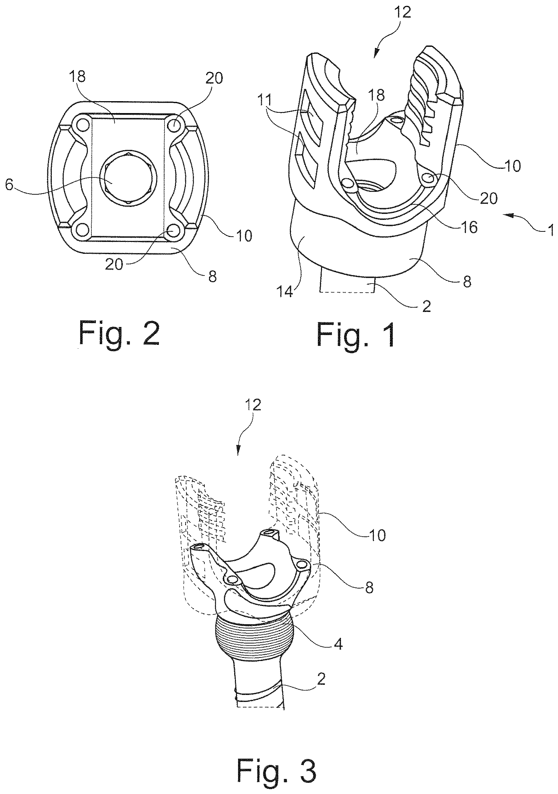

Die in den Fig. 1 bis 3 gezeigte polyaxiale Pedikelschraube 1 gemäß dem bevorzugten Ausführungsbeispiel der Erfindung hat einen mit einem Außengewinde versehenen Schaft oder Anker 2, an dessen einem proximalen Ende ein vorzugsweise sphärischer oder zumindest semi-sphärischer Schaftkopf 4 aus- oder angeformt ist. In den Schaftkopf 4 ist ein Werkzeugeingriff 6 beispielsweise für einen Imbus-Schraubenschlüssel, einen Kreuzschlitz-Schraubendreher, etc. eingearbeitet, um den Schaft 2 in den Pedikelkanal eines Wirbels (nicht dargestellt) einschrauben zu können.

Wie aus der Fig. 3 zu ersehen ist, bildet der Schaftkopf 4 an seinem Übergangsbereich zum geschraubten Schaft/Schaftabschnitt 2 eine in Axialrichtung wirkende Hinterschneidung. Diese entsteht vorliegend dadurch, indem der Schaftkopfdurchmesser größer gewählt ist als der Schaftdurchmesser. Alternativ wäre es aber auch denkbar, den Schaftkopf 4 durch eine Eindrehung (nicht gezeigt) am proximalen Endabschnitt des Schafts 2 vom übrigen geschraubten Schaftabschnitt 2 abzusetzen.

Gemäß der Fig. 3 wird der Schaftkopf 4 von einer Aufnahmehülse 8 umgriffen, die (zunächst) dreh- und schwenkbar vom Schaftkopf 4 gehalten ist.

Im Konkreten hat die Aufnahmehülse 8 einen beidseits offenen zylindrischen Hülsenkörper 10, dessen Hülsenwand von einer proximalen Stirnseite her U-förmig aufgeschlitzt ist, wodurch sich eine im Wesentlichen lotrecht zur Hülsenachse verlaufende durchgehende Querrinne (Schlitz) 12 ergibt. Die Aufnahmehülse 8 ist im Axialbereich hin zur Offenseite des U-förmigen Schlitzes 12 mit einem Innengewinde ausgebildet, das sich gemäß der Fig. 2 in etwa bis zu einem axialen Mittenabschnitt der Hülse 8 erstreckt. In dem gegenüberliegenden Axialbereich (bzw. am axial distalen Ende) der Hülse 8, d.h. axial hinter dem Schlitzgrund des U-förmigen Schlitzes 12 ist die Aufnahmehülse 8 mit einem umlaufenden inneren Radialvorsprung/Absatz 14 ausgebildet, der den Hülseninnendurchmesser stirnseitig einengt und somit eine Hinterschneidung darstellt. Der Durchmesser des inneren Radialvorsprungs 14 ist dabei so bemessen, dass er geringfügig größer ist als der des geschraubten Schaftabschnitts 2 jedoch kleiner ist als der des Schaftkopfs 4. Ferner ist der Innendurchmesser der Hülse 8 bzw. des Hülsenkörpers 10 geringfügig größer als der Durchmesser des Schaftkopfs 4. Durch die U-förmigen Längsschlitze 12 ergeben sich zwei radial gegenüberliegende Flanken im Hülsenkörper 10, an deren jeweilige äußere Mantelseite Aufnahmehülse-Eingriffselemente 11 an- oder ausgeformt sind. Hierbei handelt es sich vorliegend jeweils um zwei axial beabstandete Einkerbungen/Taschen, die in Axialrichtung wirkende Hinterschneidungen ausbilden. Alternativ hierzu ist aber genauso möglich, leistenförmige Vorsprünge an der äußeren Mantelseite der Flanken zu formen, welche die Aufnahmehülse-Eingriffselemente 11 bilden. Grundsätzlich ist daher jedwede Form von Eingriffselement denkbar sofern hierdurch eine axiale (provisorische) Zugkraft in Richtung weg vom Schaftkopf 4 auf die Aufnahmehülse 8 anlegbar ist.

In die Aufnahmehülse 8 ist ein stempelartiges Kraftübertragungsbauteil (Inlay) 16 eingesetzt/eingelegt. Dieser Stempel 16 wird im Wesentlichen durch eine Stempelhülse gebildet, die an ihrer einen Stirnseite am Schaftkopf 4 aufliegt. Die andere Stirnseite der Stempelhülse (oder einfach Stempel) 16 ist zu einem flanschartigen Kragen 18 aufgeweitet, der in Draufsicht gemäß der Fig. 2 oval oder rechteckförmig ist und in Seitenansicht (siehe auch Fig. 1) zu einer konkaven Rinne/Wanne ausgeformt ist. An den Ecken der Rechtecksform sind Stempel-Eingriffselemente bzw. (Sack-) Löcher 20 axial eingearbeitet, welche Krafteinleit- oder Angriffspunkte für ein werkzeugartiges externes/separates Hilfsmittel darstellen.

Die Aufnahmehülse 8 und der Stempel 16 sind konstruktiv so aufeinander abgestimmt, dass der Stempel 16 zumindest über eine vorbestimmte Strecke in der Aufnahmehülse 6 verschiebbar ist. Des Weiteren ist der Stempel/Bolzen 16 derart in die Aufnahmehülse 8 eingelegt, dass die stirnseitig am Stempel 16 ausgeformte/ausgewölbte Wanne am Kragen 18 die beiden parallelen U-förmigen Schlitze 12 diametral miteinander verbindet und so die Auflage für einen eingelegten Längsträger bildet, wie dies nachfolgend noch beschrieben wird.

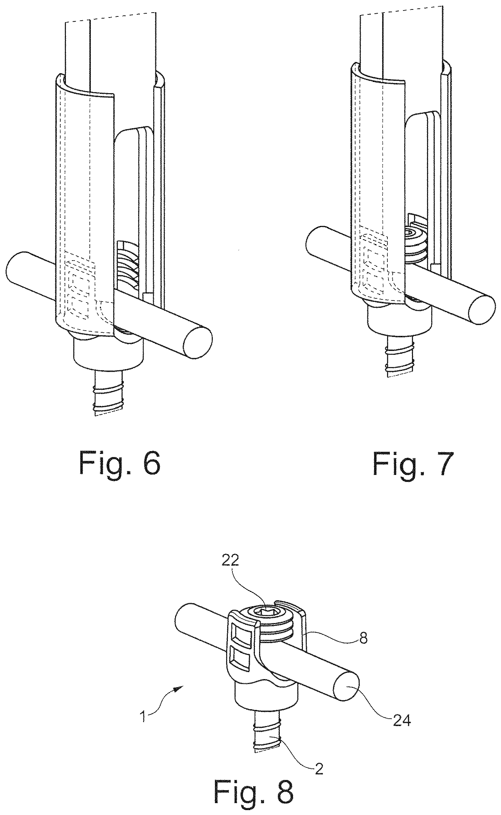

Die Funktionsweise der polyaxialen Pedikelschraube gemäß dem bevorzugten Ausführungsbeispiel der Erfindung wird nachstehend insbesondere anhand der Fig. 8 beschrieben.

Demzufolge ist die erfindungsgemäße Pedikelschraube 1 an ihren geschraubten Schaft 2 im Pedikelkanal eines Wirbels fest verankert. In das Innengewinde der Aufnahmehülse 8 ist ein Verriegelungselement vorzugsweise in Form einer Madenschraube 22 eingedreht, wodurch der/die U-förmige(n) Schlitz(e) 12 zur schaftabgewandten Stirnseite der Aufnahmehülse 8 verschlossen ist. In die hierdurch sich ausbildende laterale Durchgangsöffnung ist/wird ein Längsträger 24 eingelegt, der Pedikelschrauben unterschiedlicher (axial benachbarter) Wirbel longitudinal verbindet.

Die Madenschraube 22 ist so festgezogen, dass die Polyaxialität der Pedikelschraube 1, d.h. die Relativlage zwischen Schaft 2 und Aufnahmehülse 8 wie gewünscht fest verriegelt ist und gleichzeitig der Längsträger 24 fest zwischen dem Stempel 16 und der Madenschraube 22 eingeklemmt ist. In diesem Verriegelungszustand wird folglich die Schraubkraft der Madenschraube 22 über den Längsträger 24 und den Stempel 16 auf den Schaftkopf 4 übertragen und spannt diesen (reibschlüssig) zwischen dem Stempel 16 und dem inneren Radialvorsprung 14 der Aufnahmehülse 8 ein.

Das Innengewinde im Hülsenkörper 10 ist so bemessen, dass es eine Selbsthemmung bewirkt. Dies bedeutet, dass die Madenschraube 22 nur mit erheblichem Kraftaufwand wieder gelöst werden kann. Darüber hinaus ist die Aufnahmehülse 8 vor dem Festziehen der Madenschraube 22 gegenüber dem Schaft 2 beweglich, sodass keine Justierkräfte manuell über die Aufnahmehülse 8 auf den Schaft 2 aufbringbar sind, beispielsweise um den betreffenden Wirbel lagezuverändern.

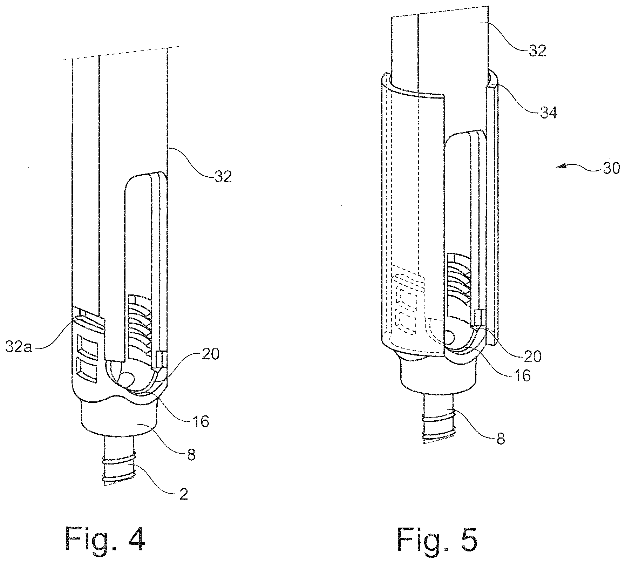

Zur Lösung dieser Problematik ist ein zusätzliches Hilfsmittel 30 gemäß der Fig. 4 bis 7 vorgesehen.

Dieses Hilfsmittel 30 besteht prinzipiell aus einem ersten Bauteil (Druckstößel) 32, das mit dem Stempel 16 in (Formschluss-) Eingriff bringbar ist und einem zweiten Bauteil (Zughülse) 34, das mit der Aufnahmehülse 8 in (Formschluss-) Eingriff bringbar ist.

Im Konkreten ist das erste Bauteil 32 des Hilfsmittels 30 gemäß der Fig. 4 ein Druckstößel oder Kolben, an dessen einer (distaler) Stirnseite eine Anzahl von Zapfen/Dornen ausgeformt sind, deren Größe und Platzierung den (Sack-) Löchern 20 im Stempel 16 entsprechen. Diese sind infolge der kreisfernen Grundrissform des Kragens 18 außerhalb der Aufstandsfläche des vorzugsweise kreisförmigen Verriegelungselements 22 angeordnet, sodass der Druckstößel 32 auch dann noch mit den (Sack-) Löchern 20 für eine provisorische/temporäre Druckkraftbeaufschlagung des Stempels 16 in Eingriff bringbar ist, wenn das Verriegelungselement 22 bereits in die Aufnahmehülse 8 eingedreht und über den zwischengelegten Längsträger 24 gegen den Stempel 16 geringfügig abgestützt ist.

Im einfachsten Fall ist der Druckstößel 32 hülsenförmig und umgreift die Aufnahmehülse 8 völlig. Eine etwas kompliziertere Formgebung gemäß der Fig. 4 sieht vor, dass der Druckstößel 32 an seinem distalen Endabschnitt zwei diametrale Axialaussparrungen 32a hat, die so bemessen sind, dass die beiden Flanken der Aufnahmehülse 8 im Wesentlich passgenau darin axial eingeführt werden können. In diesem Fall kann also der mittlere Durchmesser des Druckstößels 32 in etwa dem der Aufnahmehülse 8 entsprechen. Darüber hinaus ist der Druckstößel 32 zusätzlich U-förmig längs geschlitzt in Übereinstimmung mit den Schlitzen 12 in der Aufnahmehülse 8, sodass diese Schlitze 12 bei angesetztem Druckstößel 32 von diesem nicht verschlossen werden und somit ein Längsträger auch bei angesetztem Hilfsmittel in den Längsschlitz der Aufnahmehülse 8 eingeführt werden kann.

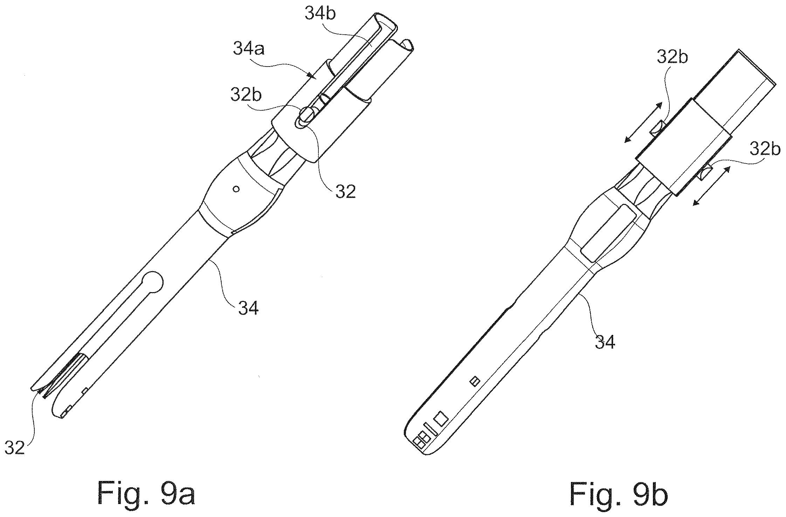

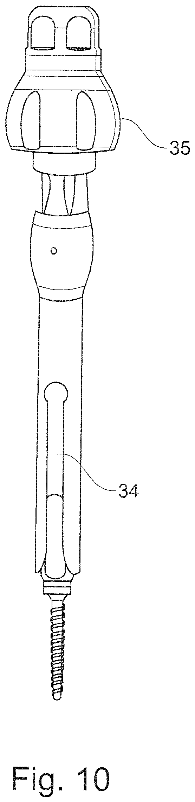

Gemäß der Fig. 5 ist um den Druckstößel 32 die Zughülse 34 relativ verschieblich angeordnet, wobei optional zwischen Druckstößel 32 und Zughülse 34 noch eine Mittel-/Zwischenhülse (nicht weiter dargestellt) angeordnet sein kann. Die Zughülse 34 hat an ihrem distalen Endabschnitt eine Anzahl von (nicht weiter dargestellten) Rastelementen, welche mit den Einkerbungen/Ausbuchtungen 11 an den Flanken des Hülsenkörpers 10 in lösbaren Rasteingriff bringbar sind. Schließlich ist die Zughülse 34 an ihrem gegenüberliegenden (proximalen) Endabschnitt mit dem Druckstößel 32 gekoppelt, derart, dass beide Bauteile über eine Kupplung gegeneinander bewegbar und damit vorspannbar sind wie sie beispielhaft in den Fig. 9 und 10 gezeigt ist.

Beispielsweise kann die Kupplung eine Wellenmutter sein, die drehbar, jedoch axialfest an der Zughülse 34 gelagert und mit dem Druckstößel 32 in Schraubeingriff ist. Wird demzufolge die Wellenmutter manuell gedreht, bewegen sich Druckstößel 32 und Zughülse 34 relativ zueinander in entgegengesetzte Richtungen. Auf diese Weise kann durch entsprechendes manuelles Drehen der Wellenmutter der Druckstößel 32 in Richtung hin zum Stempel 16 und gleichzeitig die Zughülse 34 in Richtung weg vom Stempel 16 bewegt werden. In diesem Fall kann die Aufnahmehülse 8 gegenüber dem Stempel 16 provisorisch/temporär über das beschriebene Hilfsmittel 30 verspannt werden, wobei der dazwischen angeordnete Schaftkopf 4 ebenfalls provisorisch/temporär eingeklemmt wird.

Alternativ hierzu ist es aber auch möglich, die Zughülse 34 gemäß der Fig. 9 an ihrem proximalen, der Pedikelschraube abgewandten Endabschnitt mit einem Außengewinde 34a auszubilden, wobei die Zughülse 34 an diesem proximalen Endabschnitt mit zumindest einem Längsschlitz 34b (vorzugsweise zwei diametrale Längsschlitze) versehen ist. In diesem Fall hat der Drückstößel 32 an seinem proximalen Ende radial vorragende Mitnehmerstifte 32b, welche durch die Längsschlitze 34b an der Zughülse 34 radial nach Außen vorstehen. Gemäß der Fig. 10 ist auf das Außengewinde 34a der Zughülse 34 eine Wellenmutter 35 aufgeschraubt, die mit den Mitnahmestiften 32b des Druckstößels 32 in Eingriff ist. Wird nunmehr die Wellenmutter 35 auf der Zughülse 34 gedreht, wird hierdurch eine Relativbewegung zwischen dem Druckstößel 32 und der Zughülse 34 erzeugt.

Abschließend sei an dieser Stelle darauf hingewiesen, dass auch andere Kupplungsformen denkbar sind, die unter Ausnutzung einer Hebelwirkung bzw. einer Untersetzung eine Relativbewegung zwischen Druckstößel 32 und Zughülse 34 bewirken.

Die Funktionsweise des erfindungsgemäßen Hilfsmittels 30 wird nachfolgend anhand der Fig. 4 bis 7 näher beschrieben.

Demzufolge wird zunächst gemäß der Fig. 4 der Druckstößel 32 in die (Sack-) Löcher 20 des Stempels/Bolzens 16 eingeführt, wobei die Flanken am Hülsenkörper 10 in den Axialaussparrungen 32a des Druckstößels 32 längs geführt werden. In dieser Relativposition überlappen sich die Schlitze 12 an der Aufnahmehülse 8 mit dem U-förmigen Schlitz im Druckstößel 32, wodurch sich eine Art Lateralfenster ausbildet.

Anschließend wird die Zughülse 34 über den Druckstößel 32 geführt und mit ihren Rastvorsprüngen in den Einkerbungen 11 an der Aufnahmehülse 8 verrastet. Auch die Zughülse 34 ist, wie in der Fig. 5 deutlich erkennbar ist, mit U-förmigen Schlitzen in Übereinstimmung mit dem Schlitz im Druckstößel 32 versehen, sodass bei übergezogener Zughülse 34 das Lateralfenster weiterhin geöffnet bleibt.

Sobald das Hilfsmittel 30 am Stempel 16 aufsitzt und mit der Aufnahmehülse 8 verrastet ist, wird die beispielsweise in den Fig. 9 und 10 gezeigte Kupplung (Wellenmutter) 35 betätigt und somit der Schaftkopf 4 provisorisch/temporär verspannt. Je nach Art der Kupplung sind hierbei provisorische Vorspannkräfte erreichbar, durchaus ähnlich der Vorspannkräfte des eigentlichen Verriegelungselements der Pedikelschraube.

In diesem Zustand kann nunmehr eine Kraft ausgehend vom Hilfsmittel 30 über die Aufnahmehülse 8 auf den Schaft 2 übertragen werden, um beispielsweise den Wirbel zu justieren. Hierauf wird gemäß der Fig. 6 der Längsträger 24 in das Lateralfenster eingelegt und das Verriegelungselement, vorzugsweise die vorab bereits eingesetzte Madenschraube 22 festgezogen, ohne dass das Hilfsmittel 30 vorher entfernt werden muss. Mit dem Festziehen der Madenschraube 22 wird die Polyaxialität endgültig verriegelt, sodass das Hilfsmittel 30 hierauf abgenommen werden kann. Damit ist der Montagevorgang beendet.

Abschließend sei die vorliegende Erfindung nochmals wie Folgt zusammengefasst: Offenbart wird eine polyaxiale Pedikelschraube 1 mit einem geschraubten Schaftabschnitt 2 zur Verankerung der Pedikelschraube 1 in einem Wirbel, an dessen einem Axialende ein Schaftkopf 4 ausgebildet ist, der mit einer Aufnahmehülse 8 für einen Längsträger 24 dreh- und/oder schwenkbar sowie Zugkraft übertragend gekoppelt ist. Die Aufnahmehülse 8 weist ein Fixiermittel zur wahlweisen Lagefixierung der Aufnahmehülse 8 bezüglich des Schaftabschnitts 2 auf, zumindest bestehend aus einem in der Aufnahmehülse 8 gelagerten sowie auf den Schaftkopf 4 einwirkenden Stempel/Bolzen 16 und einem auf den Stempel 16 einwirkenden Verriegelungselement 22. Der Stempel (Inlay) 16 ist mit vom Verriegelungselement 22 nicht abgedeckten oder überlagerten Stempel-Eingriffselementen 20 ausgebildet/versehen für das provisorische/temporäre Einleiten einer Druckkraft auf den Stempel 16 parallel zum Verriegelungselement 22.

Des Weiteren ist ein werkzeugartiges Hilfsmittel 30 offenbart, das (parallel zum Verriegelungselement 22) mit dem Stempel/Bolzen 16 an dessen Stempel-Eingriffselementen 20 für eine provisorische/temporäre Druckkraftbeaufschlagung und mit der Aufnahmehülse 8 vorzugsweise an deren Eingriffselementen 11 für eine provisorische/temporäre Zugkraftbeaufschlagung ansetzbar ist. Vorzugsweise hat das Hilfsmittel 30 zwei Bauteile, nämlich ein Druck- 32 und ein Zugbauteil 34, die so miteinander gekoppelt sind, dass eine provisorische Druckbeaufschlagung des Stempel 16 über das eine Bauteil zwangsläufig zu einer Zugbelastung der Aufnahmehülse 8 über das andere Bauteil zum Erreichen eines Kräftegleichgewichts führt. Optional ist das werkzeugartige Hilfsmittel 30 zumindest teilweise als Ein-Weg-Produkt vorgesehen. Insbesondere das Druckbauteil 32 sollte bevorzugt als Ein-Weg-Bauteil ausgebildet sein, da dies vergleichsweise nahe an den Wirbel eines Patienten gelangt und somit ggf. mit Körperflüssigkeit kontaminiert wird. Grundsätzlich ist jedoch günstig, wenn das gesamte Hilfsmittel 30 nach seiner einmaligen Verwendung entsorgt wird.

Schließlich besteht ein Stabilisierungssystem gemäß der Erfindung aus der polyaxialen Pedikelschraube 1, dem Hilfsmittel 30 und vorzugsweise einem Längsträger 24, der in die Aufnahmehülse 8 eingelegt und festgeklemmt wird.

## Patentansprüche

1. Polyaxiale Pedikelschraube mit einem geschraubten Schaftabschnitt (2) zur Verankerung der Pedikelschraube (1) in einem Wirbel, an dessen einem Axialende ein Schaftkopf (4) ausgebildet ist, der mit einer Aufnahmehülse (8) für einen Längsträger (24) dreh- und/oder schwenkbar gekoppelt ist, welche ein Fixiermittel zur wahlweisen Lagefixierung der Aufnahmehülse (8) bezüglich des Schaftabschnitts (2) aufweist, zumindest bestehend aus einem in der Aufnahmehülse (8) gelagerten sowie auf den Schaftkopf (4) einwirkenden Stempel (16) und einem über den Längsträger (24) auf den Stempel (16) einwirkenden, sowie unmittelbar in die Aufnahmehülse (8) ein- oder aufgedrehten Verriegelungselement (22), wobei der Stempel (16) mit vom Verriegelungselement (22) nicht abgedeckten oder überlagerten Stempel-Eingriffselementen (20) ausgebildet ist für das Einleiten einer provisorischen Druckkraft auf den Stempel (16) parallel zum Verriegelungselement (22) und die Aufnahmehülse (8) einen beidseits offenen, insbesondere zylindrischen, Hülsenkörper (10) aufweist, dessen Hülsenwand von einer proximalen Stirnseite her U-förmig aufgeschlitzt ist, wodurch sich zwei radial gegenüberliegende Flanken ergeben, **dadurch gekennzeichnet, dass** sich die Stempel-Eingriffselemente (20) zwischen beiden Flanken befinden.

2. Polyaxiale Pedikelschraube nach Anspruch 1, **dadurch gekennzeichnet, dass** der Stempel (16) derart ausgeformt ist, dass er zumindest abschnittsweise radial über das Verriegelungselement (22) vorsteht bzw. freiliegt, wodurch an diesen radialen Abschnitten, die vom Verriegelungselement (22) nicht abgedeckt oder überlagert sind, die Stempel-Eingriffselemente (20) entstehen.

3. Polyaxiale Pedikelschraube nach Anspruch 2, **dadurch gekennzeichnet, dass** der Stempel (16) zumindest an einem axialen Abschnitt einen kreisabweichenden Grundriss, vorzugsweise einen ovalen Grundriss, hat, wodurch Grundrissabschnitte mit großem Durchmesser radial über das Verriegelungselement (22) vorragen und die Stempel-Eingriffselemente (20) enthalten.

4. Polyaxiale Pedikelschraube nach einem der Ansprüche 2 und 3, **dadurch gekennzeichnet, dass** die Stempel-Eingriffselemente (20) durch axiale Zentrierlöcher markiert sind, die zur Aufnahme und Platzierung eines externen Hilfsmittels (30) zur provisorischen Druckbeaufschlagung des Stempels (16) dienen.

5. Polyaxiale Pedikelschraube nach einem der vorstehenden Ansprüche, **dadurch gekennzeichnet, dass** die Aufnahmehülse (8) mit zumindest einem Aufnahmehülse-Eingriffselement (11) ausgebildet oder versehen ist, über das eine äußere provisorische Zugkraft auf die Aufnahmehülse (8) parallel zum Verriegelungselement (22) aufbringbar ist.

6. Polyaxiale Pedikelschraube nach Anspruch 5, **dadurch gekennzeichnet, dass** das Aufnahmehülse-Eingriffselement (11) eine Hinterschneidung, vorzugsweise an der äußeren Mantelseite der Aufnahmehülse (8) und insbesondere in Form von zwei axial beabstandeten Einkerbungen / Taschen, ausformt.

7. Hilfsmittel zur provisorischen Fixierung der Polyaxialität einer Pedikelschraube (1) gemäß einem der Ansprüche 1 bis 6, bestehend aus einem geschraubten Schaft (2) mit Schaftkopf (4), der von einer Aufnahmehülse (8) umgeben ist, in der ein durch ein Verriegelungselement (22) gegen den Schaftkopf (4) vorspannbarer Stempel (16) gelagert ist, **gekennzeichnet durch** ein erstes Bauteil (32) für das drückende in Eingriff kommen unmittelbar mit am Stempel (16) vorgesehenen und vom Verriegelungselement (22) nicht abgedeckten oder nicht überlagerten Stempel-Eingriffselemente (20), und ein zweites Bauteil (34), das relativ, vorzugsweise gegenläufig, zum ersten Bauteil (32) bewegbar ist und ein Rastmittel hat, das mit der Aufnahmehülse (8), vorzugsweise an deren Aufnahmehülse-Eingriffselement (11), in ziehenden Eingriff bringbar ist.

8. Hilfsmittel nach Anspruch 7, **dadurch gekennzeichnet, dass** das erste Bauteil (32) in Form eines Druckstößels und das zweite Bauteil (34) in Form eines Zugbauteils ausgebildet ist.

9. Hilfsmittel nach Anspruch 8, **dadurch gekennzeichnet, dass** das Zugbauteil (34) eine den Druckstößel (32) umgebende Hülse ist, an deren einem Axialende radial einwärts vorstehende Rastvorsprünge ausgeformt sind und die mit dem Druckstößel (32) über eine Kupplung wirkverbunden ist.

10. Hilfsmittel nach Anspruch 9, **dadurch gekennzeichnet, dass** die Kupplung ein Hebelmechanismus ist, vorzugsweise bestehend aus einem an einem der Bauteile aus Druckstößel (32) oder Hülse (34) anscharnierten Hebel, der über ein Gelenk mit dem jeweils anderen Bauteil gekoppelt ist.

11. Hilfsmittel nach Anspruch 9, **dadurch gekennzeichnet, dass** die Kupplung eine relativ an der Hülse (34) drehbare, jedoch axialfest gehaltene Wellenmutter ist, die mit dem Druckstößel (32) in Schraubeingriff steht **oder dass** die Kupplung eine Wellenmutter (35) ist, die mit einem Gewinde (34a) am Druckstößel (32) in Schraubeingriff steht sowie mit einem bezüglich der Hülse (34) axial verschiebbaren, drehfesten Mitnehmer (32b) gekoppelt ist, der an dem Druckstößel (32) fixiert ist.

12. Hilfsmittel nach einem der Ansprüche 8 bis 11, **dadurch gekennzeichnet, dass** zumindest der Druckstößel (32) mit einem vorzugsweise U-förmigen Längsschlitz von der Kontaktseite mit dem Stempel (16) her versehen ist, wodurch zwei Flanken entstehen, an denen sich der Druckstößel (32) an der Stirnseite des Stempels (16) aufstützt.

13. Stabilisierungssystem das folgende Bestandteile hat:
eine polyaxiale Pedikelschraube (1) gemäß einem der Ansprüche 1 bis 6, bestehend aus einem geschraubten Schaft (2) mit Schaftkopf (4), der von einer Aufnahmehülse (8) umgeben ist, in der ein durch ein Verriegelungselement (22) gegen den Schaftkopf (4) vorspannbarer Stempel (16) gelagert ist, und
ein manuell betätigbares Hilfsmittel (30) gemäß einem der Ansprüche 7 bis 12, das ein erstes Bauteil (32) zur wahlweisen provisorischen und unmittelbaren Druckbeaufschlagung von vom Verriegelungselement (22) nicht abgedeckten oder nicht überlagerten Stempel-Eingriffselementen (20) des Stempels (16), und ein zweites Bauteil (34) zur wahlweisen provisorischen Zugbelastung der Aufnahmehülse (8) jeweils parallel zum Verriegelungselement (22) hat.

14. Stabilisierungssystem nach Anspruch 13, **gekennzeichnet durch** einen Längsträger (24), der in einem Querschlitz (12) der Aufnahmehülse (8) einlegbar ist, wobei zumindest das erste Bauteil (32), und vorzugsweise auch das zweite Bauteil (34), des Hilfsmittels (30) so gestaltet ist, dass es den Längsträger (24) umgreift.

15. Polyaxiale Pedikelschraube nach einem der Ansprüche 1 bis 6, **dadurch gekennzeichnet, dass** die Stempel-Eingriffselemente (20) als Formschluss-Eingriffselemente, vorzugsweise in Form von Axiallöchern, ausgebildet sind.

## Claims

1. Polyaxial pedicle screw having a screwed shaft section (2) for anchoring the pedicle screw (1) in a vertebra, wherein a shaft head (4) is configured at an axial end of the shaft section (2), said shaft head (4) being pivotably and/or rotatingly coupled to a receiving sleeve (8) for a longitudinal support (24), wherein the receiving sleeve (8) comprises a fastening means for selective positional fastening of the receiving sleeve (8) with respect to the shaft section (2), said fastening means at least comprising an inlay (16) mounted in the receiving sleeve (8) and acting on the shaft head (4), and a locking element (22) acting on the inlay (16) via the longitudinal support (24) and screwed directly into the receiving sleeve (8), wherein the inlay (16) is configured with inlay engagement elements (20) which are not covered or overlapped by the locking element (22), for the introduction of a provisional compressive force parallel to the locking element (22) on the inlay (16) and the receiving sleeve (8) has an, in particular, cylindrical sleeve body (10) open on both sides, whose sleeve wall is slotted in a U-shape on the proximal end face, whereby there occur two flanks lying radially opposite each other, **characterized in that** the inlay engagement elements (20) are positioned between the two flanks.

2. Polyaxial pedicle screw according to claim 1, **characterized in that** the inlay (16) is formed such that it projects or is exposed, at least in portions, radially over the locking element (22), whereby the inlay engagement elements (20) occur at these radial portions which are not covered or overlapped by the locking element (22).

3. Polyaxial pedicle screw according to claim 2, **characterized in that** the inlay (16) has an outline deviating from a circle, preferably an oval outline, at least at one axial portion whereby outline portions of large diameter protrude radially over the locking element (22) and contain the inlay engagement elements (20).

4. Polyaxial pedicle screw according to one of claims 2 and 3, **characterized in that** the inlay engagement elements (20) are marked by axial centring holes which serve for receiving and placement of an external helping means (30) for the provisional application of pressure to the inlay (16).

5. Polyaxial pedicle screw according to one of the above claims, **characterized in that** the receiving sleeve (8) is configured or provided with at least one receiving-sleeve engagement element (11) via which an external provisional tensile force parallel to the locking element (22) can be applied to the receiving sleeve (8).

6. Polyaxial pedicle screw according to claim 5, **characterized in that** the receiving-sleeve engagement element (11) forms an undercut preferably on the external sleeve-side of the receiving sleeve (8) and in particular in the form of two axially spaced notches/pockets.

7. Helping means for the provisional fastening of the polyaxiality of a pedicle screw (1), according to any one of claims 1 to 6 consisting of a screwed shaft (2) having a shaft head (4) which is surrounded by a receiving sleeve (8) in which an inlay (16) is mounted which is biasable against the shaft head (4) by means of a locking element (22), **characterized by** a first member (32) for coming into direct pressing engagement with inlay engagement elements (20) provided at the inlay (16) and which are not covered or overlapped by the locking element (22), and a second member (34), which is movable relative to, preferably in opposition to the first member (32), and has a latching means which can be brought into pulling engagement with the receiving sleeve (8), preferably at its receiving-sleeve engagement element (11).

8. Helping means according to claim 7, **characterized in that** the first member (32) is formed in the form of a plunger and the second member (34) in the form of a pulling member.

9. Helping means according to claim 8, **characterized in that** the pulling member (34) is a sleeve surrounding the plunger (32), wherein at an axial end of the sleeve, radially inwardly protruding latching projections are formed and wherein the sleeve is operatively connected with the plunger (32) via a coupling.

10. Helping means according to claim 9, **characterized in that** the coupling is a lever mechanism preferably consisting of a hinged lever on one of the members among the plunger (32) and the sleeve (34), wherein the lever is coupled via a joint to the respective other member.

11. Helping means according to claim 9, **characterized in that** the coupling is a shaft-nut rotatably but also axially fixedly held relative to the sleeve (34) and is in screwed engagement with the plunger (32) **or that** the coupling is a shaft-nut (35) which is in screwed engagement with a thread (34a) on the plunger (32) and is coupled to a rotationally fixed carrier (32b) which is axially moveable relative to the sleeve (34), wherein the carrier (32b) is fixed to the plunger (32).

12. Helping means according to any one of claims 8 to 11, **characterized in that** at least the plunger (32) is provided with a preferably U-shaped longitudinal slot starting from the contact side with the inlay (16), whereby two flanks occur at which the plunger (32) rests on the end face of the inlay (16).

13. Stabilization system that has the following components: a polyaxial pedicle screw (1), according to any one of claims 1 to 6 consisting of a screwed shaft (2) having a shaft head (4) surrounded by a receiving sleeve (8) in an inlay (16) which is mounted which is biasable against the shaft head (4) by means of a locking element (22), and a manually operatable helping means (30), according to any one of claims 7 to 12, which has a first member (32) for the selective provisional and direct application of pressure to inlay engagement elements (20) of the inlay (16) which are not covered or overlapped by the locking element (22), and a second member (34) for the selective provisional tensile loading of the receiving sleeve (8), which is parallel to the locking element (22) in each case.

14. Stabilization system according to claim 13, **characterized by** a longitudinal member (24) which is insertable in a transverse slot (12) of the receiving sleeve (8), wherein at least the first member (32) and preferably also the second member (34) of the helping means (30) is designed such that it engages around the longitudinal member (24).

15. Polyaxial pedicle screw according to any one of claims 1 to 6, **characterized in that** the inlay engagement elements (20) are configured as engagement elements (20) having form-locking fit, preferably in the form of axial holes.

## Revendications

1. Vis pédiculaire polyaxiale comportant une partie de tige (2) vissée assurant l'ancrage de la vis pédiculaire (1) dans une vertèbre, à une extrémité axiale de laquelle est réalisée une tête de tige (4) qui est couplée de manière à pouvoir tourner et/ou pivoter avec un manchon de logement (8) pour un support longitudinal (24), lequel comporte un moyen de fixation permettant la fixation sélective du manchon de logement (8) par rapport à la partie de tige (2), composé au moins d'un poinçon (16) monté dans le manchon de logement (8) et agissant sur la tête de tige (4), ainsi qu'un élément de verrouillage (22) agissant sur le poinçon (16) par l'intermédiaire du support longitudinal (24) et vissé ou dévissé directement dans le manchon de logement (8), dans laquelle le poinçon (16) est composé d'éléments d'engrènement de poinçon (20) non recouverts ou chevauchés par l'élément de verrouillage (22) pour l'introduction d'une force de pression temporaire sur le poinçon (16) parallèlement à l'élément de verrouillage (22) et le manchon de logement (8) comporte un corps de manchon (10) ouvert des deux côtés, en particulier cylindrique, dont la paroi de manchon est fendue en forme de U depuis une face frontale proximale, conduisant à deux flancs radialement opposés, **caractérisé en ce que** les éléments d'engrènement de poinçon (20) se trouvent entre les deux flancs.

2. Vis pédiculaire polyaxiale selon la revendication 1, **caractérisée en ce que** le poinçon (16) est formé de telle sorte qu'il est en saillie et/ou exposé au moins en partie de manière radiale au-dessus de l'élément de verrouillage (22), moyennant quoi, au niveau de ces parties radiales qui ne sont pas recouvertes ou chevauchées par l'élément de verrouillage (22), apparaissent les éléments d'engrènement de poinçon (20).

3. Vis pédiculaire polyaxiale selon la revendication 2, **caractérisée en ce que** le poinçon (16) possède au moins au niveau d'une partie axiale un plan différent d'un cercle, de préférence un plan ovale, moyennant quoi des sections de plan avec un grand diamètre font saillie radialement au-dessus de l'élément de verrouillage (22) et comportent les éléments d'engrènement de poinçon (20).

4. Vis pédiculaire polyaxiale selon l'une quelconque des revendications 2 et 3, **caractérisée en ce que** les éléments d'engrènement de poinçon (20) sont marqués par des trous de centrage axiaux, lesquels servent à recevoir et placer un moyen auxiliaire externe (30) pour la mise sous pression temporaire du poinçon (16).

5. Vis pédiculaire polyaxiale selon l'une quelconque des revendications précédentes, **caractérisée en ce que** le manchon de logement (8) est formé ou pourvu d'au moins un élément d'engrènement de manchon de logement (11), par l'intermédiaire duquel une force de traction externe temporaire peut être appliquée sur le manchon de logement (8) parallèlement à l'élément de verrouillage (22).

6. Vis pédiculaire polyaxiale selon la revendication 5, **caractérisée en ce que** l'élément d'engrènement de manchon de logement (11) forme une contre-dépouille, de préférence au niveau de l'enveloppe externe du manchon de logement (8) et en particulier sous forme de deux encoches/poches éloignées axialement.

7. Moyen auxiliaire permettant la fixation temporaire de la polyaxialité d'une vis pédiculaire (1) selon l'une quelconque des revendications 1 à 6, comportant une tige (2) vissée avec une tête de tige (4), qui est entourée par un manchon de logement (8), dans lequel est monté un poinçon (16) pouvant être précontraint par un élément de verrouillage (22) contre la tête de tige (4), **caractérisé par** un premier composant (32) pour l'engrènement par pression directement avec des éléments d'engrènement de poinçon (20) prévus sur le poinçon (16) et non recouverts ou non chevauchés par l'élément de verrouillage (22), et un second composant (34) qui est mobile de manière relative, de préférence en sens opposé, par rapport au premier composant (32) et possède un moyen d'encliquetage qui peut être amené en engrènement par traction avec le manchon de logement (8), de préférence au niveau de son élément d'engrènement de manchon de logement (11).

8. Moyen auxiliaire selon la revendication 7, **caractérisé en ce que** le premier composant (32) est réalisé en forme de poussoir et le second composant (34) est réalisé en forme de composant de traction.

9. Moyen auxiliaire selon la revendication 8, **caractérisé en ce que** le composant de traction (34) est un manchon entourant le poussoir (32), à une extrémité axiale duquel sont formées des saillies d'encliquetage faisant saillie radialement vers l'intérieur et qui est relié fonctionnellement avec le poussoir (32) via un dispositif d'accouplement.

10. Moyen auxiliaire selon la revendication 9, **caractérisé en ce que** le dispositif d'accouplement est un mécanisme de levier, de préférence composé d'un levier fixé par charnière au niveau de l'un des composants parmi le poussoir (32) ou le manchon (34), qui est couplé avec l'autre composant respectif par l'intermédiaire d'une articulation.

11. Moyen auxiliaire selon la revendication 9, **caractérisé en ce que** le dispositif d'accouplement est un écrou d'arbre pouvant tourner de manière relative au niveau du manchon (34), mais maintenu de manière fixe axialement, lequel se trouve en engrènement vissé avec le poussoir (32) ou **en ce que** le dispositif d'accouplement est un écrou d'arbre (35) qui se trouve en engrènement vissé avec un filetage (34a) au niveau du poussoir (32) et est couplé avec un élément d'entraînement (32b) fixe en rotation et mobile axialement par rapport au manchon (34), qui est fixé au poussoir (32).

12. Moyen auxiliaire selon l'une quelconque des revendications 8 à 11, **caractérisé en ce qu'**au moins le poussoir (32) est pourvu d'une fente longitudinale de préférence en forme de U depuis le côté de contact avec le poinçon (16), conduisant à deux flancs au niveau desquels le poussoir (32) s'appuie sur la face frontale du poinçon (16).

13. Système de stabilisation qui possède les composants suivantes :
une vis pédiculaire polyaxiale (1) selon l'une quelconque des revendications 1 à 6, comportant une tige (2) vissée avec une tête de tige (4), qui est entourée par un manchon de logement (8), dans lequel est monté un poinçon (16) pouvant être précontraint par un élément de verrouillage (22) contre la tête de tige (4), et
un moyen auxiliaire (30) pouvant être actionné manuellement selon l'une quelconque des revendications 7 à 12, qui possède un premier composant (32) pour la mise sous pression sélective temporaire et directe d'éléments d'engrènement de poinçon (20) du poinçon (16) non recouverts ou non chevauchés par l'élément de verrouillage (22), et un second composant (34) pour la charge de traction sélective temporaire du manchon de logement (8) de manière respectivement parallèle à l'élément de verrouillage (22).

14. Système de stabilisation selon la revendication 13, **caractérisé par** un support longitudinal (24) qui peut être inséré dans une fente transversale (12) du manchon de logement (8), dans lequel au moins le premier composant (32), et de préférence également le second composant (34), du moyen auxiliaire (30) est conçu de telle sorte qu'il entoure le support longitudinal (24).

15. Vis pédiculaire polyaxiale selon l'une quelconque des revendications 1 à 6, **caractérisée en ce que** les éléments d'engrènement de poinçon (20) sont réalisés sous forme d'éléments d'engrènement par complémentarité de formes, de préférence sous forme de trous axiaux.
